# EUROPÄISCHE PATENTSCHRIFT

(11) **EP 0 472 678 B1**
(45) Veröffentlichungstag und Bekanntmachung des Hinweises auf die Patenterteilung: **31.07.1996**
(21) Anmeldenummer: 91903636.8
(22) Anmeldetag: 02.02.1991
(51) Int. Cl.: C12N 9/02, C12Q 1/26, C12N 9/96

(54) **NITRATREDUKTASE AUS HEFEN, IHRE HERSTELLUNG UND VERWENDUNG**
NITRATE REDUCTASE DERIVED FROM YEASTS, ITS PRODUCTION AND USE
REDUCTASE DES NITRATES PROVENANT DE LEVURES, SA FABRICATION ET SON UTILISATION

(30) Priorität: 16.02.1990 DE 4004900
(43) Veröffentlichungstag der Anmeldung: 04.03.1992
(73) Patentinhaber: MERCK PATENT GmbH, D-64271 Darmstadt (DE)
(72) Erfinder: JOHANNSSEN, Walther, D-6107 Reinheim (DE); SCHWARTZ, Harry, D-6238 Hofheim-Diedenbergen (DE); GROMES, Reiner, D-6114 Gross-Umstadt (DE); HEINRICH, Martin, D-6100 Darmstadt (DE)
(86) Internationale Anmeldenummer: EP9100199
(87) Internationale Veröffentlichungsnummer: WO9112319

(56) Entgegenhaltungen:
- EP-A- 0 244 771
- Chemical Abstracts, Band 105, Nr. 25, 22. Dezember 1986 (Columbus, Ohio, US) P. V. Choudary et al.: "Partial purification and properties of the assimilatory nitrate reductase of the food yeast Candida utilis.", siehe Seite 333
- Chemical Abstracts, Band 85, Nr. 19, 8. November 1976 (Columbus, Ohio, US) V.P. Choudary et al.: "Characterization of nitrate reductase from the yeast Candida utilis", siehe Seite 194

## Beschreibung

Die Erfindung betrifft NAD(P)H-abhängige Nitratreduktase aus Hefen, ein Verfahren zu ihrer Herstellung und ihre Verwendung in einem Reagenz zur Bestimmung von Nitrat.

Die quantitative Bestimmung von Nitrat in den unterschiedlichsten Proben, z.B. in Wässern (Mineralwasser, Leitungswasser, Abwasser), Lebensmitteln (Obst, Gemüse, Bier, Wein, Säfte, Fleisch und Fleischerzeugnisse, Milch und Milchprodukte, Babynahrung) und Bodenproben gewinnt zunehmend an Bedeutung. Nitratbestimmungen sind insbesondere bei Verdacht auf gesundheitsschädliche, überhöhte Xonzentrationen von großer Wichtigkeit, um beispielsweise eine potentielle cancerogene Nitrosaminbildung verhindern zu können.

Es sind sowohl physikalische (HPLC, Gelchromatographie) als auch chemische Methoden zur Bestimmung der Nitratkonzentration bekannt. Bei den Üblichen chemischen Methoden wird das Nitrat zu Nitrit reduziert und dieses über eine Diazotierungsreaktion nachgewiesen. Diese Verfahren sind sehr aufwendig und können bei Bestimmungen in vielen organischen Materialien nicht problemlos angewendet werden.

Aus der EP 244 771 ist eine stabilisierte Nitratreduktase und ein Reagenz zur Bestimmung von Nitrat mit Hilfe dieser Nitratreduktase bekannt. Das Reagenz enthält neben dem Enzym und NAD(P)H, einen zwitterionischen Puffer und einen Stickstoffheterocycluspuffer. Das Enzym hat jedoch den Nachteil, daß seine Herstellung sehr aufwendig ist, und daß es eine störende Nitritreduktase-Aktivität zeigt, wodurch die beschriebene vollenzymatische Nitratbestimmung problematisch ist. Außerdem hat sich gezeigt, daß die in der EP 244 771 beschriebene Nitratreduktase Nitrat zu Nitrit nur mit dem relativ teueren NADPH befriedigend umsetzt, nicht jedoch mit NADH.

Der Erfindung lag die Aufgabe zugrunde, eine Nitratreduktase zu finden und zu isolieren, die stabil genug ist, und mit der es möglich ist, Nitrat auch in geringen Konzentrationen mit einem einfachen, kostengünstigen und schnellen Test genau zu bestimmen.

Überraschenderweise hat sich gezeigt, daß Hefen, die mit Nitrat als einziger Stickstoffquelle in einem vollsynthetischen Nährmedium unter aeroben Bedingungen wachsen können, über eine Nitratreduktase verfügen, die sich mit einem einfachen Verfahren isolieren läßt und die stabilisiert werden kann. Mit der so hergestellten Nitratreduktase läßt sich eine Nitratbestimmung durchführen, die die Nachteile der bekannten Methoden vermeidet.

Gegenstand der Erfindung ist eine NAD(P)H-abhängige Nitratreduktase enthaltendes Gemisch, das gekennzeichnet ist durch ein mit Hilfe der Gelpermeationschromatographie ermitteltes Molekulargewicht der Nitratreduktase im nativen Zustand von ca. 350 000 D und das dadurch erhältlich ist, daß man Nitratreduktase von Typ EC 1.6.6.2 enthaltende, in einem vollsynthetischen Nährmedium mit Nitrat als einziger Stickstoffquelle kultivierte Hefezellen in Phosphatpuffer, pH 6,5-8,5, aufschließt, den Rohextrakt an einem Anionenaustauscher chromatographiert, die Nitratreduktase enthaltenden Fraktionen mit 10-50 % Protein versetzt, durch Ultrafiltration aufkonzentriert und durch Wirbelschichtgranulierung trocknet.

Ein weiterer Gegenstand der Erfindung ist ein Verfahren zur Herstellung einer NAD(P)H-abhängigen Nitratreduktase enthaltenden Gemisches, das dadurch gekennzeichnet ist, daß man in einem vollsynthetischen Nährmedium mit Nitrat als einziger Stickstoffquelle kultivierte Hefezellen in Phosphatpuffer, pH 6,5-8,5, in Gegenwart eines Komplexbildners, Mercaptoethanol, FAD, Kaliumhexacyanoferrat und einem Proteaseinhibitor aufschließt, nach Zentrifugation den Rohextrakt an einem Anionenaustauscher direkt chromatographiert, die Nitratreduktase enthaltenden Fraktionen mit 10-50 % Protein versetzt, durch Ultrafiltration aufkonzentriert und durch Wirbelschichtgranulierung trocknet, sowie ein Reagenz zur Bestimmung von Nitrat, das gekennzeichnet ist durch einen Gehalt an Nitratreduktase enthaltendem Gemisch, NAD(P)H und einem Farbreagenz zur Bestimmung von Nitrit.

Als geeignete Hefen zur Herstellung der erfindungsgemäßen NAD(P)H-abhängigen Nitratreduktase kommen z.B. solche der Gattungen Candida, Hansenula und Williopsis infrage, vorzugsweise die Gattungen Candida und Hansenula und die Organismen Candida boidinii (DSM 70 026), Hansenula ciferrii (DSM 70 780), Hansenula saturnus (DSM 70 278), insbesondere Candida boidinii.

Die Herstellung der Nitratreduktase erfolgt aus Zellen, die in einem vollsynthetischen Nährmedium mit Nitrat als einziger Stickstoffquelle angezogen werden. Der Aufschluß der Zellen erfolgt in einer Glasperlenmühle in Kaliumphosphatpuffer, in Gegenwart eines Komplexbildners (z.B. EDTA), Mercaptoethanol, Flavinadenindinucleotid (FAD), Kaliumhexacyanoferrat und eines Proteaseinhibitors (z.B. PMSF); auch andere dem Fachmann bekannte Proteaseinhibitoren können eingesetzt werden. Nach Aufschluß der Zellen und Abtrennung der Zelltrümmer durch Zentrifugation kann der so erhaltene Rohextrakt überraschenderweise ohne vorhergehende Nucleinsäure- oder fraktionierte Proteinfällung an einem Anionenaustauschermaterial (z.B. einem weitporigen, vernetzten Vinylpolymer mit aufgepfropften Polykationen und Trimethylammoniummethylgruppen wie Fractogel EMD TMAE-650) chromatographiert werden, das mit Kaliumphosphatpuffer äquilibriert wurde. Die aktive Nitratreduktase wird mit einem ansteigenden KCl-Gradienten bei ca. 140 mmol/l KCl eluiert. Ein im Stand der Technik genannter Zusatz von Zucker oder Zuckeralkoholen ist für eine Enzymstabilisierung während der Aufreinigung nicht notwendig. Die Fraktionen, die Nitratreduktase-Aktivität enthalten, werden vereinigt, mit 10-50 % Rinderserumalbumin oder anderen Proteinen (z.B. Collagenhydrolysate) versetzt und durch Ultrafiltration aufkonzentriert. Die so erhaltene Lösung enthält durchschnittlich 6-7 Units Nitratreduktase/mg Protein und wird anschließend durch eine Wirbelschichtgranulierung getrocknet.

Das vollsynthetische Nährmedium, in dem die Mikroorganismen angezogen werden, enthält im wesentlichen die folgenden Bestandteile:

| | |
|---|---|
| 0,04 g/l | m-Inosit, |
| 1,0 g/l | Kaliumdihydrogenphosphat, |
| 1,0 g/l | Magnesiumsulfat, |
| 4,5 g/l | Kaliumhydrogentartrat, |
| 1,5 g/l | Kaliumnitrat, |
| 20,0 g/l | Glucose. |

Die nach dem geschilderten Verfahren hergestellte Nitratreduktase, insbesondere eine solche aus Candida boidinii, hat folgende K_{M}-Werte:

| | |
|---|---|
| NADH | 7,2 . 10⁻⁵ M |
| NADPH | 6,9 . 10⁻⁵ M |
| KNO₃(NADH) | 4,0 . 10⁻³ M |
| KNO₃(NADPH) | 4,0 . 10⁻⁴ M. |

Das pH-Optimum in Phosphatpuffer liegt bei 7,0 (NADH-abhängig) bzw. 7,1 (NADPH-abhängig). Das Temperaturoptimum ist bei 30 °C (NADH-abhängig) und 25 °C (NADPH-abhängig). Das Molekulargewicht im nativen Zustand, bestimmt mit Gelpermeationschromatographie, beträgt ca. 350 000 D. Eine Endprodukthemmung durch Nitrit erfolgt bei der NADH-abhängigen Nitratreduktase nur bis 50 %.

Zur Herstellung des erfindungsgemäßen Reagenzes zur Bestimmung von Nitrat hat es sich als besonders vorteilhaft herausgestellt, wenn man das Enzym durch Aufsprühen der Lösung auf vorgeformte Seeds und anschließende Einkapselung mit Polyvinylpyrrolidon in eine stabile trockene Form bringt. Die Seeds können z.B. aus Mannit, Polyethylenglykol oder Polyvinylpyrrolidon bestehen (US 4,820,627). Das so hergestellte Enzymgranulat zeichnet sich durch eine sehr geringe Restfeuchtigkeit eine gute Lagerstabilität und sehr gute Löslichkeit aus. Eine Tablettierung des Granulates mit reproduzierbar einstellbarer Enzymaktivität pro Tablette erhöht die Anwenderfreundlichkeit. Das erfindungsgemäße Enzympräparat ist bei 25 °C in 50 mmol/l Kaliumphosphatpuffer, pH 7,0 über mehrere Stunden stabil. Als Granulat ist das Nitratreduktase-Präparat bei einer Lagerung von -20 °C praktisch unbegrenzt haltbar.

Mit Hilfe dieser stabilisierten Nitratreduktase wird Nitrat zu Nitrit reduziert und anschließend das entstandene Nitrit in einer chemischen Nachweisreaktion durch Diazotierung z.B. mit Sulfanilsäure und N-Naphthylethylendiammoniumchlorid bei 540 oder 525 nm bestimmt. Die für die Bestimmung erforderlichen Reagenzlösungen enthalten außer 0,1-1 U, vorzugsweise 0,7 U Nitratreduktase noch 50-200 mmol/l, vorzugsweise 100 mmol/l Kaliumphosphatpuffer, pH 7, 100-1000 µmol/l, vorzugsweise 400 µmol/l FAD, 200-600 µmol/l, vorzugsweise 400 µmol/l Kaliumhexacyanoferrat und 3,5-5 mmol/l, vorzugsweise 4 mmol/l NADH oder NADPH. Mit dem erfindungsgemäßen Reagenz ist es möglich, Nitrat über einen weiten Konzentrationsbereich (0,5 bis > 100 mg/l Nitrat) quantitativ in den unterschiedlichsten Matrices, teilweise ohne Probenvorbereitung, zu bestimmen. Unter Einbeziehung von Nitrat-Standardlösungen ist die Erstellung einer Eichkurve möglich. Es ist nicht notwendig, die zu bestimmende Nitratmenge vollständig zu reduzieren. Eventuell störende Nebenaktivitäten des Enzympräparates wie z.B. Nitritreduktase-Aktivitäten und "NAD(P)H-Oxidase"-Aktivitäten spielen bei dem beschriebenen kinetischen Verfahren ebenfalls keine Rolle. Damit steht nun ein biochemisches Reagenz zur quantitativen Bestimmung von Nitrat zur Verfügung. Ein weiterer Vorteil des erfindungsgemäßen Reagenzes liegt in der Verwendungsmöglichkeit von NADH anstelle des sehr teuren und weniger gut löslichen NADPH. Die quantitative Bestimmung von Nitrat ist mit dem beschriebenen Verfahren innerhalb weniger Minuten bei Raumtemperatur durchführbar. Andere biochemische Verfahren benötigen deutlich längere Analysenzeiten.

### Beispiel 1

### Herstellung von Nitratreduktase

a) Kultur und Ernte der Zellen
   Hefezellen (Candida boidinii, DSM 70 026) zur Gewinnung von Nitratreduktase werden über die Stufen 10-ml-Schüttelröhrchen, 200-ml-Erlenmeyerkolben, 10-l-Fermenter, 100-l-Fermenter, in einem vollsynthetischen Medium mit Kaliumnitrat als alleiniger Stickstoffquelle kultiviert. Nach einer Laufzeit von 26 Stunden im 100-l-Fermenter werden die Zellen vom Medium getrennt und mit kaltem Puffer A gewaschen. (Puffer A: Kaliumphosphatpuffer, 0,1 mol/l, pH-Wert 7,5, enthaltend 1 mmol/l EDTA, 0,1 mmol/l Mercaptoethanol, 4 µmol/l FAD, 20 µmol/l Kaliumhexacyanoferrat. Die Ausbeute beträgt 8,6 kg Hefezellen (Frischgewicht).
   Das vollsynthetische Medium ist wie folgt zusammengesetzt: 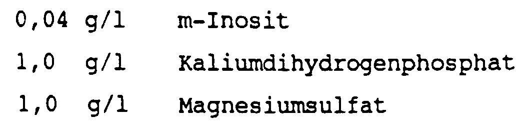 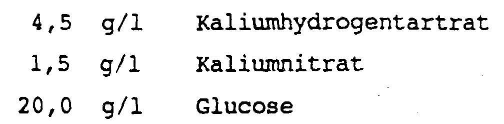
   Spurenelemente: AlCl₃ 2 mg/l, H₃BO₃ 2 mg/l, CuSO₄ . 5 H₂O 1 mg/l, FeCl₃ . 6 H₂O 2 mg/l, KH-Tartrat 20 mg/l, KJ 1 mg/l, Na₂MoO₄ . 2 H₂O 2 mg/l, ZnSO₄ . 7 H₂O 2 mg/l
   Vitamine: 4-Aminobenzoesäure 0,2 mg/l, Biotin 0,02 mg/l, Folsäure 0,02 mg/l, Nicotinsäure 1 mg/l, Ca-D-Panthothenat 1 mg/l, Pyridoxoliumchlorid 1 mg/l, Riboflavin 0,5 mg/l, Thiamindichlorid 0,5 mg/l. Der pH-Wert wird mit Natronlauge auf 5,0 eingestellt.
b) Herstellung zellfreier Extrakte
   2,5 kg der so gewonnenen Zellen werden in 4,5 1 Puffer B (Puffer A + 0,1 mmol/l Protease-Inhibitor, z.B. PMSF) suspendiert und in einer Glasperlenmühle aufgeschlossen. Bedingungen des Aufschlusses: Glaskugeln Durchmesser 0,5-0,7 mm, Glaskugelfüllung 80-85 %, 2000 rpm, Durchsatz 10 l/h, Temperatur während des Aufschlusses < 10 °C. Die Zelltrümmer werden anschließend durch Zentrifugation (15000 x g/30 min) abgetrennt. In 4,5 1 Rohextrakt wird eine Gesamt-Volumenaktivität von 40 kU und eine spezifische Aktivität von 420 mU NADH-abhängige Nitratreduktase pro mg Protein gemessen.
c) Reinigung der Nitratreduktase durch Anionenaustauscherchromatographie
   Der im vorhergehenden Schritt erhaltene Rohextrakt wird mit Puffer B bis auf eine Leitfähigkeit < 8 mS verdünnt und ohne Zwischenschaltung einer Nucleinsäurefällung oder einer fraktionierten Neutralsalzfällung direkt an einer Anionenaustauschersäule (Fractogel EMD TMAE-650(M)) chromatographiert. Die Säule wird zuvor mit Puffer B äquilibriert. Aktive Nitratreduktase wird mit einem ansteigenden KCl-Gradienten in Puffer B im Bereich von 140 mmol/l KCl eluiert. Aktive Fraktionen werden vereinigt, mit 10-50 % Rinderserumalbumin oder vergleichbaren Proteinen versetzt und die Nitratreduktase durch Ultrafiltration an einer Membran (Ausschlußgrenze 30 000 Dalton) aufkonzentriert. Die so erhaltene Lösung hat ein Volumen von 500 ml und eine spezifische Nitratreduktase-Aktivität von 7 U/mg Protein. Die Gesamtaktivität beträgt 23 kU (Ausbeute 57 %). Vergleichbare Ausbeuten erhält man mit Hefezellen aus Hansenula ceferrii (DSM 70 780) und Hansenula saturnus (DSM 70 278) als Ausgangsmaterialien.
d) Wirbelschichtgranulierung und Tablettierung
   Die aufkonzentrierte Nitratreduktase-Fraktion wird im Wirbelschicht-Granulationsverfahren auf einen vorbereiteten Träger (Mannit/Polyethylenglycol/Polyvinylpyrrolidon) aufgebracht. Das so hergestellte Enzymgranulat (2,4 kg) enthält 7 Units Nitratreduktase pro Gramm Granulat. Das Granulat wird ohne weitere Zusätze in Tablettenform gepreßt und bei -20 °C gelagert.

### Beispiel 2

Verfahren zur Bestimmung von Nitrat

### Reagenzien:

1) Reagenzmischung:
   50 mmol/l Kaliumphoshatpuffer, pH 7,0
   20 µmol/l FAD
   20 µmol/l K₃[Fe(CN)₆]
   in wirbelschichtgranulierter Form
2) NADH-Tabletten nach Wirbelschichtgranulierung auf Mannit-Seeds (1,413 mg NADH pro Tablette)
3) Nitratreduktase-Tabletten nach Wirbelschichtgranulierung (0,7 Units Nitratreduktase/Tablette)
4) Nitrit-Farbreagenz:
   97 mg Sulfanilsäure,
   3 mg N-(1-Naphthyl)-ethylendiammoniumchlorid
5) Nitrat-Standardlösung (100 mg/l)

Vorbereiten der Probelösungen:
1. Zur Bestimmung des Blindwertes:
Als Probelösung dient demineralisiertes Wasser.
2. Zur Aufstellung einer Eichkurve:
Die 100 mg/l Nitrat enthaltende Standardlösung wird mit demineralisiertem Wasser zu den gewünschten Eichlösungen verdünnt.
Variante 1 enthält 20/40/60/80/100 mg/l Nitrat-Standard Variante 2 enthält 2/4/6/8/10 mg/l Nitrat-Standard
Die Proben werden vor der Messung klar filtriert und gegebenenfalls mit demineralisiertem Wasser verdünnt.
Herstellen der Reaktionslösung (für 10 Einzelbestimmungen):

| Reagenz-Nr. | Variante 1 (5-100 mg/l) | Variante 2 (0,5-10 mg/l) |
|---|---|---|
| 1 | 100 mg | 200 mg |
| 2 | 1 Tablette | 1 Tablette |
| 3 | 1 Tablette | 1 Tablette |
| lösen in deminerasiertem Wasser: | 9 ml | 15 ml |

Durchführung der Bestimmung:

| Bedingungen/Einzelbestimmung | Variante 1 (5-100 mg/l) | Variante 2 (0,5-10 mg/l) |
|---|---|---|
| Reaktionslösung | 0,9 ml | 1,5 ml |
| Start der Reaktion durch Zugabe von Probelösung | 0,1 ml | 0,5 ml |
| Reaktionszeit | 7 min | ca. 20 min |
| Abstoppen der Reaktion durch Zugabe von Reagenz 4 | 100 mg | 100 mg |
| Verdünnung mit Wasser | + 2 ml | keine |

Nach dem Absetzen von Reagenz 4 erfolgt die Bestimmung der Extinktionen bei 540 nm oder 525 nm aus dem Überstand von Standards und der Proben gegen den Leerwert.

### Auswertung:

a) mittels einer Eichkurve
b) E_{Probe} : E_{Standard} = Konzen._{Probe} : Konzen._{Standard}

### Beispiel 3

Trinkwasserproben werden direkt zum Test eingesetzt. Bierproben und andere Kohlendioxid enthaltenden Getränke werden zur Entfernung des Kohlendioxids geschüttelt. Fruchtsäfte und andere trübe Proben werden zentrifugiert und filtriert.

5 g Milch werden mit 20 ml 50%iger Trichloressigsäure versetzt, mit Natronlauge auf pH 7 eingestellt, in einen 100-ml-Meßkolben überführt und mit Wasser aufgefüllt. Nach dem Zentrifugieren und Filtrieren wird das klare Filtrat zum Test eingesetzt.

Etwa 5 g Fleich- oder Fleichprodukte der gut homogenisierten Probe werden in ein Becherglas eingewogen, mit 20 ml 50%iger Trichloressigsäure-Lösung und 30 ml Wasser versetzt und im Mixer nochmals homogenisiert. Anschließend wird mit Natronlauge (2 mol/l) auf pH 7,0 eingestellt, 5 ml Petroleumbenzin zugegeben und nach Mischen in einen 100-ml-Meßkolben überführt. Mit Wasser wird soweit aufgefüllt, bis die Petroleumbenzin-Schicht vollständig über der Marke liegt. Nach sorgfältigem Mischen und nach Phasentrennung wird die Petroleumbenzin-Schicht abgesaugt.

Die wäßrige Phase wird durch ein Faltenfilter filtriert und zum Test eingesetzt.

Zur Probenvorbereitung bei Gemüse, Früchten und Salat wird ein Preßsaft hergestellt, der anschließend zentrifugiert und filtriert wird. Eine entsprechend mit Wasser verdünnte Lösung wird zum Test eingesetzt.

Analog Beispiel 2 werden mit den so vorbereiteten Proben Nitratbestimmungen durchgeführt. Typische Meßwerte und die Wiederfindungsraten sind in der folgenden Tabelle enthalten.

| Probe | Nitratkonzentration (mg/l) | Wiederfindung (%) (Zumischversuch mit 50 % (v/v) Probe, 50 % (v/v) Standard |
|---|---|---|
| Kartoffeln | 270 | 99 |
| Zwiebeln | 24 | 102 |
| Paprika, gelb | 35 | 95 |
| Gurken | 41 | 96 |
| Bier | 7 | 96 |
| Mineralwasser | 2 | 99 |
| Ananassaft | 11 | 101 |
| Orangensaft | 5 | 101 |
| Kopfsalat | 1700 | 96 |

## Patentansprüche

1. NAD(P)H-abhängige Nitratreduktase enthaltendes Gemisch, gekennzeichnet durch ein mit Hilfe der Gelpermeationschromatographie ermitteltes Molekulargewicht der Nitratreduktase im nativen Zustand von ca. 350 000 D und dadurch erhältlich, daß man Nitratreduktase vom Typ EC 1.6.6.2 enthaltende, in einem vollsynthetischen Nährmedium mit Nitrat als einziger Stickstoffquelle kultivierte Hefezellen in Phosphatpuffer, pH 6,5-8,5, aufschließt, den Rohextrakt an einem Anionenaustauscher chromatographiert, die Nitratreduktase enthaltenden Fraktionen mit 10-50 % Protein versetzt, durch Ultrafiltration aufkonzentriert und durch Wirbelschichtgranulierung trocknet.

2. NAD(P)H-abhängige Nitratreduktase enthaltendes Gemisch nach Anspruch 1, gekennzeichnet durch folgende K_{M}-Werte:
| | |
|---|---|
| NADH | 7,2 · 10⁻⁵ M |
| NADPH | 6,9 · 10⁻⁵ M |
| KNO₃(NADH) | 4,0 · 10⁻³ M |
| KNO₃(NADPH) | 4,0 · 10⁻⁴ M. |

3. NAD(P)H-abhängige Nitratreduktase enthaltendes Gemisch nach Anspruch 1, dadurch gekennzeichnet, daß das vollsynthetische Nährmedium im wesentlichen folgende Bestandteile enthält:
| | |
|---|---|
| 0,04 g/l | m-Inosit, |
| 1,0 g/l | Kaliumdihydrogenphosphat, |
| 1,0 g/l | Magnesiumsulfat, |
| 4,5 g/l | Kaliumhydrogentartrat, |
| 1,5 g/l | Kaliumnitrat, |
| 20,0 g/l | Glucose. |

4. Verfahren zur Herstellung eines NAD(P)H-abhängigen Nitratreduktase enthaltenden Gemisches, dadurch gekennzeichnet, daß man in einem vollsynthetischen Nährmedium mit Nitrat als einziger Stickstoffquelle kultivierte Hefezellen in Phosphatpuffer, pH 6,5-8,5, in Gegenwart eines Komplexbildners, Mercaptoethanol, FAD, Kaliumhexacyanferrat und einem Proteaseinhibitor aufschließt, nach Zentrifugation den Rohextrakt an einem Anionenaustauscher direkt chromatographiert, die Nitratreduktase enthaltenden Fraktionen mit 10-50 % Protein versetzt, durch Ultrafiltration aufkonzentriert und durch Wirbelschichtgranulierung trocknet.

5. Verfahren nach Anspruch 4, dadurch gekennzeichnet, daß als Hefezellen solche der Gattungen Candida und Hansenula verwendet werden.

6. Verfahren nach den Ansprüchen 4 und 5, dadurch gekennzeichnet, daß als Hefezellen solche aus Candida boldinii verwendet werden.

7. Reagenz zur Bestimmung von Nitrat, gekennzeichnet durch einen Gehalt an Nitratreduktase enthaltendem Gemisch nach Anspruch 1, NAD(P)H und einem Farbreagenz zur Bestimmung von Nitrit.

## Claims

1. Mixture containing NAD(P)H-dependent nitrate reductase, characterized by a molecular weight of the nitrate reductase in the native state of about 350 000 D, determined with the aid of gel permeation chromatography, and obtainable by yeast cells which have been cultivated in a completely synthetic nutrient medium with nitrate as the sole nitrogen source and which contain nitrate reductase of the EC 1.6.6.2 type being disrupted in phosphate buffer, pH 6.5-8.5, the crude extract being chromatographed on an anion exchanger, the fractions containing nitrate reductase being mixed with 10-50 % protein, concentrated by ultrafiltration and dried by fluidized bed granulation.

2. Mixture containing NAD(P)H-dependent nitrate reductase according to Claim 1, characterized by the following K_{M} values:
| | |
|---|---|
| NADH | 7.2 × 10⁻⁵ M |
| NADPH | 6.9 × 10⁻⁵ M |
| KNO₃ (NADH) | 4.0 × 10⁻³ M |
| KNO₃ (NADPH) | 4.0 × 10⁻⁴ M. |

3. Mixture containing NAD(P)H-dependent nitrate reductase according to Claim 1, characterized in that the completely synthetic nutrient medium contains essentially the following constituents

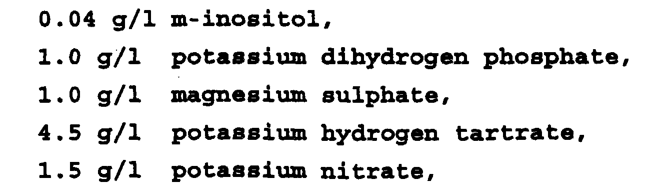

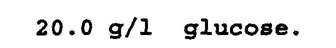

4. Process for the preparation of a mixture containing NAD(P)H-dependent nitrate reductase, characterized in that yeast cells which have been cultivated in a completely synthetic nutrient medium with nitrate as the sole nitrogen source are disrupted in phosphate buffer, pH 6.5-8.5, in the presence of a complexing agent, mercaptoethanol, FAD, potassium hexacyanoferrate and a protease inhibitor, after centrifugation the crude extract is directly chromatographed on an anion exchanger, the fractions containing nitrate reductase are mixed with 10-50 % protein, concentrated by ultrafiltration and dried by fluidized bed granulation.

5. Process according to Claim 4, characterized in that the yeast cells used are of the genera Candida and Hansenula.

6. Process according to Claims 4 and 5, characterized in that the yeast cells used are of Candida boidinii.

7. Reagent for determining nitrate, characterized by a content of mixture containing nitrate reductase according to Claim 1, NAD(P)H and a colour reagent for determining nitrite.

## Revendications

1. Mélange contenant de la nitrate réductase dépendant du NAD(P)H, caractérisé par un poids moléculaire de la nitrate réductase à l'état natif d'environ 350 000 D, déterminé à l'aide de la chromatographie de perméation des gels, et que l'on peut obtenir en attaquant des cellules de levure contenant de la nitrate réductase du type EC 1.6.6.2., cultivées dans un milieu nutritif entièrement synthétique avec du nitrate comme unique source d'azote, dans un tampon phosphaté à pH 6,5-8,5, en chromatographiant l'extrait brut sur un échangeur d'anions, en mélangeant les fractions contenant la nitrate réductase avec 10-50% de protéine, en concentrant par ultrafiltration et en séchant par granulation sur une couche fluidisée.

2. Mélange contenant de la nitrate réductase dépendant du NAD(P)H selon la revendication 1, caractérisé par les valeurs de K_{M} suivantes:
| | |
|---|---|
| NADH | 7,2 x 10⁻⁵ M |
| NADPH | 6,9 x 10⁻⁵ M |
| KNO₃ (NADH) | 4,0 x 10⁻³ M |
| KNO₃ (NADPH) | 4,0 x 10⁻⁴ M. |

3. Mélange contenant de la nitrate réductase dépendant du NAD(P)H selon la revendication 1, caractérisé en ce que le milieu nutritif entièrement synthétique contient essentiellement les composants suivants:
| | |
|---|---|
| 0,04 g/l | de m-inositol, |
| 1,0 g/l | de phosphate diacide de potassium, |
| 1,0 g/l | de sulfate de magnésium, |
| 4,5 g/l | de tartrate acide de potassium, |
| 1,5 g/l | de nitrate de potassium, |
| 20,0 g/l | de glucose. |

4. Procédé de préparation d'un mélange contenant de la nitrate réductase dépendant du NAD(P)H, caractérisé en ce qu'on attaque des cellules de levure cultivées dans un milieu nutritif entièrement synthétique avec du nitrate comme unique source d'azote, dans un tampon phosphaté à pH 6,5-8,5 , en présence d'un agent complexant, de mercaptoéthanol, de FAD, d'hexacyanoferrate de potassium et d'un inhibiteur de protéase, en ce qu'après centrifugation on chromatographie directement l'extrait brut sur un échangeur d'anions, en ce qu'on mélange les fractions contenant la nitrate réductase avec 10-50% de protéine, en ce qu'on concentre par ultra-filtration et en ce qu'on sèche par granulation sur une couche fluidisée.

5. Procédé selon la revendication 4, caractérisé en ce qu'on utilise comme cellules de levure des cellules des genres Candida et Hansenula.

6. Procédé selon les revendications 4 et 5, caractérisé en ce qu'on utilise comme cellules de levure des cellules de Candida boidinii.

7. Réactif de détermination du nitrate, caractérisé en ce qu'il contient un mélange contenant de la nitrate réductase selon la revendication 1, du NAD(P)H et un réactif coloré pour la détermination du nitrite.
